# EUROPEAN PATENT APPLICATION

(11) **EP 4 678 106 A1**
(43) Date of publication of application: **14.01.2026**
(21) Application number: 24187007.0
(22) Date of filing: 08.07.2024
(51) Int. Cl.: A61B 6/00

(54) **CEILING-SUSPENDED X-RAY HOLDING SYSTEM**

(71) Applicant: Koninklijke Philips N.V., 5656 AG Eindhoven (NL)
(72) Inventor: DUTT, Tara, Eindhoven (NL); GHUMRE, Milind, Eindhoven (NL)
(74) Representative: Philips Intellectual Property & Standards

(57) **Abstract**

The present invention relates to a ceiling-suspended holding system for an X-ray tube head assembly. The holding system comprises a stationary rail system configured to be mounted to a ceiling of an X-ray examination room, a movable rail system configured to be mounted to the stationary rail system, wherein the movable rail system is configured to move along the stationary rail system in a first direction, a carriage configured to be mounted to the movable rail system, wherein the carriage is configured to move along the movable rail system in a second direction, and a manipulator comprising at least a first elongate member and a second elongate member. The first elongate member is configured to be mounted with a first end of the first elongate member to the carriage, and with a second end of the first elongate member to a first end of the second elongate member, and the second elongate member is configured to be mounted with a second end of the second elongate member to an X-ray tube head assembly. The manipulator comprises a first hinge in-between the first elongate member and the second elongate member.

## Description

### FIELD OF THE INVENTION

The present invention relates to a ceiling-suspended holding system for an X-ray tube head assembly, and an X-ray device comprising the ceiling-suspended holding system.

### BACKGROUND OF THE INVENTION

In medical imaging, ceiling-suspended X-ray systems typically comprise a horizontal movable carriage, which is able to move in longitudinal (x-axis) and transverse (y-axis) directions parallel to the ceiling. These motions are possible over long extrusion rails of desired length as per clinical workflow.

For the different clinical use cases, the heavy X-ray tube head assembly has to be moved vertically up and down along the z-axis. Therefore, a telescopic column assembly can be attached to the carriage at the top side and holds the X-ray tube head of a medical imaging system at the bottom end of the telescopic column. Thus, vertical movement of the X-ray tube head along the z-axis is possible by the extension and contraction of the telescoping column. The X-ray tube head assembly can be counter balanced by a spring assembly or some sort of energy storing device mounted on the horizontally moving carriage.

As at least five-axis movement of the X-ray tube head is required for clinical workflows, the X-ray tube head can usually be rotated in the horizontal plane around a swing/swivel axis, and in the vertical plane around the tilt axis with respect to the telescopic column. The swing axis and tilt axis movements can be provided by separate hardware mechanisms.

Thus, the ceiling-suspended support system has to be bulky, heavy, and robust enough to take the load of the X-ray tube assembly and allow the movements of X-ray tube assembly during the intended service life of the X-ray device.

In addition, electrical cables for electrical connection of the X-ray tube assembly have to be guided to the tube, usually through a flexible corrugated tube to allow for horizontal and vertical movement. This corrugated tube is typically fixed at several positions on the ceiling rails and on the horizontally moving carriage. These cables can obstruct the positioning of the X-ray tube head due to the geometry of conventional ceiling suspension design.

The inventors of the present invention have thus found that it would be advantageous to have an improved ceiling-suspended holding system for an X-ray tube head assembly, which enables unobstructed movement of an X-ray tube head assembly, in particular during vertical movement or rotation around the swing axis.

### SUMMARY OF THE INVENTION

It is an object of the present invention to provide an improved ceiling-suspended holding system for an X-ray head assembly that is easy to use and provides a larger unobstructed movement range.

The object of the present invention is solved by the subject matter of the independent claims, wherein further embodiments are incorporated in the dependent claims.

The described embodiments similarly pertain to the ceiling-suspended holding system for an X-ray tube head assembly, and the X-ray device comprising the ceiling-suspended holding system. The embodiments described further may be combined in any possible way. Synergistic effects may arise from different combinations of the embodiments although they might not be described in detail.

Further on, it shall be noted that all embodiments of the present invention concerning a method might be carried out with the order of the steps as described, nevertheless this has not to be the only and essential order of the steps of the method. The herein presented methods can be carried out with another order of the disclosed steps without departing from the respective method, unless explicitly mentioned to the contrary hereinafter.

According to a first aspect of the invention, there is provided a ceiling-suspended holding system for an X-ray tube head assembly. The holding system comprises a stationary rail system configured to be mounted to a ceiling of an X-ray examination room, a movable rail system configured to be mounted to the stationary rail system, wherein the movable rail system is configured to move along the stationary rail system in a first direction, a carriage configured to be mounted to the movable rail system, wherein the carriage is configured to move along the movable rail system in a second direction, and a manipulator comprising at least a first elongate member and a second elongate member. The first elongate member is configured to be mounted with a first end of the first elongate member to the carriage, and with a second end of the first elongate member to a first end of the second elongate member, and the second elongate member is configured to be mounted with a second end of the second elongate member to an X-ray tube head assembly, and the manipulator comprises a first hinge in-between the first elongate member and the second elongate member.

Thus, the present disclosure proposes a ceiling-suspended holding system comprising fixed ceiling rails that can be connected to fixed ceiling structure, a moving ceiling rail that can be moved along the fixed ceiling rails in the x-direction, and a moving carriage preferably with bearings, that is configured to move in the y-direction with respect to moving ceiling rail. A manipulator, which can be configured to hold an X-ray tube head, is connected to the horizontally moving carriage. The manipulator can be, for example, a robotic arm, i.e., and includes serially interconnected, elongate supporting members with hinged and rotating joints to allow rotation of the manipulator around a vertical swing axis and vertical movement of a lower end of the manipulator to which an X-ray tube head assembly can be attached. At least two elongate members of the manipulator are interconnected by first hinged joint in-between. The X-ray tube head assembly can be connected as end effector to the manipulator, such that the X-ray tube head can rotate around a tilt axis.

The proposed ceiling-suspended holding system can advantageously be applied to diagnostic X-ray devices in which the X-ray source, i.e., the X-ray tube and collimator assembly, is mounted to the robotic arm which in-turn is suspended from the ceiling structures like rails. The robotic arm as manipulator provides smooth and accurate vertical movement of X-ray tube head. Also, the hinged and rotary joints between the interconnected links or members provide the degree of freedom for the rotation of the X-ray tube head with respect to the vertical axis, i.e., the swing axis rotation, and the horizontal axis, i.e., the tilt axis rotation.

Thus, the robotic arm structure with serially interconnected supporting member with hinged and rotating joints attached to a horizontally moving carriage can advantageously replace the bulky conventional telescopic arm with its counterweight mechanism of ceiling-suspended diagnostic X-ray devices. Controlled motion between the interconnected links can be initiated manually, preferably servo assisted, or automatically. The independently moving components are easily controllable by the user.

In an embodiment of the invention, the holding system comprises a second hinge between the carriage and the first elongate member of the manipulator.

In an embodiment of the invention, the holding system comprises a first rotary joint between the carriage and the first elongate member of the manipulator, the first rotary joint configured for allowing a rotation of the manipulator around a vertical axis.

In an embodiment of the invention, the manipulator comprises a second rotary joint and/or a third hinge between the second elongate member of the manipulator and the X-ray tube head assembly.

Thus, preferably, the manipulator can comprise a first rotating joint by which the manipulator is connected to the carriage, and which allows rotation around a vertical axis perpendicular to the horizontal x- and y-directions. A second hinged joint of the manipulator can be positioned between the first rotating joint and the first elongate member of the manipulator. By combined motion of the first hinge and the second hinge, a vertical movement of the lower end of the manipulator to which the X-ray tube can be attached can be performed. Preferably, a second rotating joint and a third hinged joint are arranged between the second elongate member of the manipulator and the X-ray tube head assembly.

In an embodiment of the invention, the first elongate member comprises a first unchangeable length and the second elongate member comprises a second unchangeable length.

In an embodiment of the invention, the holding system and/or the manipulator does not comprise a telescopic mechanism.

Thus, the manipulator comprises as movable elements solely joints allowing a rotary movement around an axis. In particular, the manipulator comprises hinged joints and rotary joints, where the hinged joints connecting two members allow rotation about an axis essentially perpendicular to a connection direction of the two members, and where the rotary joints connecting two members allow rotation about an axis essentially parallel to a connection direction of the two members. Therefore, the holding system and/or the manipulator, with the exception of the rails systems, does not comprise any telescopic connectors, which allow a linear moment in any direction.

In an embodiment of the invention, the manipulator comprises at least a third elongate member in-between the first and second elongate members.

In an embodiment of the invention, the third elongate member comprises a third unchangeable length.

In an embodiment of the invention, the first direction is perpendicular to the second direction, and/or the first direction and the second direction are parallel to the ceiling of the X-ray examination room.

In an embodiment of the invention, the holding system further comprises an energy chain configured for guiding electrical cables from the carriage along the manipulator to the X-ray tube head assembly. Thus, a manipulator for the vertical movement of the X-ray tube head assembly with an energy chain for cable management is proposed. The energy chain is preferably used with a manipulator; thus, the present invention makes it possible to use the energy chain for cable management in the vertical direction, thereby avoiding the issue of the X-ray tube's hindrance during movement.

In an embodiment of the invention, the stationary rail system comprises a first stationary rail and a second stationary rail, wherein the first stationary rail is parallel to the second stationary rail.

In an embodiment of the invention, the X-ray tube head assembly is an X-ray source configured to be positioned movably in the X-ray examination room.

In an embodiment of the invention, the holding system comprises a user interface and/or a force torque sensor configured for receiving positioning input of a user, a controller, and at least one actuator configured for positioning the holding system according to the positioning input of the user. The user interface and/or the force torque sensor can thus sense a force direction of input provided by the user.

Thus, the holding system can have a user interface for the radiographer to interact with the holding system. This user interface can be, for example, attached to the manipulator, and is preferably attached to the X-ray tube head assembly. The user interface can comprise a display or any other input device, and preferably comprises a force torque sensor configured for measuring for example six axes, for detecting a user force applied to the X-ray tube head assembly in different directions, and in turn can provide input how to reposition the holding system and the X-ray tube head assembly mounted thereto. A controller can be configured for receiving this input and for providing control signals to motors or actuators of the holding system for moving the joins of the manipulator and/or the rails system.

In an embodiment of the invention, the holding system further comprises a locking mechanism configured for locking the holding system in a current position.

Thus, if the locking mechanism is activated, brakes may be applied to the joints for preventing any movement of the holding system.

According to another aspect of the invention, there is provided an X-ray device comprising an X-ray tube head assembly, an X-ray detector, and a ceiling-suspended holding system according to any one of the preceding embodiments.

Thus, the benefits provided by any of the above aspects equally apply to all of the other aspects and vice versa.

In summary, the invention relates to a ceiling-suspended holding system for an X-ray tube head assembly. The holding system comprises a stationary rail system configured to be mounted to a ceiling of an X-ray examination room, a movable rail system configured to be mounted to the stationary rail system, wherein the movable rail system is configured to move along the stationary rail system in a first direction, a carriage configured to be mounted to the movable rail system, wherein the carriage is configured to move along the movable rail system in a second direction, and a manipulator comprising at least a first elongate member and a second elongate member. The first elongate member is configured to be mounted with a first end of the first elongate member to the carriage, and with a second end of the first elongate member to a first end of the second elongate member, and the second elongate member is configured to be mounted with a second end of the second elongate member to an X-ray tube head assembly. The manipulator comprises a first hinge in-between the first elongate member and the second elongate member.

One of the advantages of embodiments of the present invention is that the combined robotic arm with the ceiling mounted rail system is suitable for all radiographic examinations with ceiling-suspended diagnostic X-ray devices, allows long movement ranges, and thus enables a better workspace at low cost with a simple and sleek holding system. Also, the room aesthetic can be improved by a better product look and feel. Compared to current telescopic columns design of holding systems, electrical cables for power supply, high voltage, and control movements do not any more restrict the vertical movements of the X-ray tube head assembly, and thus do not cause interruptions during the clinical workflows. Further, inaccurate positioning issues due to conventional feedback sensors can be avoided via highly accurate positioning sensors like optical encoders used in the robotic arm drives of the manipulator.

These advantages are non-limiting and other advantages may be envisioned within the context of the present application.

The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a holding system for an X-ray head of the prior art.
Fig. 2 shows a schematic setup of a ceiling-suspended holding system for an X-ray tube head assembly according to an embodiment of the invention.
Fig. 3 shows a side view of a ceiling-suspended holding system for an X-ray tube head assembly according to an embodiment of the invention.
Fig. 4 shows a schematic setup of a ceiling-suspended holding system for an X-ray tube head assembly according to an embodiment of the invention.
Fig. 5 shows two different configurations of a ceiling-suspended holding system for an X-ray tube head assembly according to an embodiment of the invention.

### DETAILED DESCRIPTION OF EMBODIMENTS

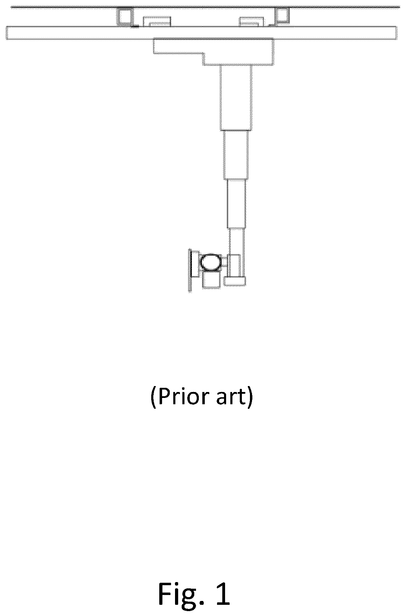

Fig. 1 shows a holding system for an X-ray head of the prior art. An X-ray tube head assembly is mounted via a telescopic mechanism to a rail system that is attached to the ceiling. However, this telescopic holding system is very bulky and heavy and lacks flexible usability.

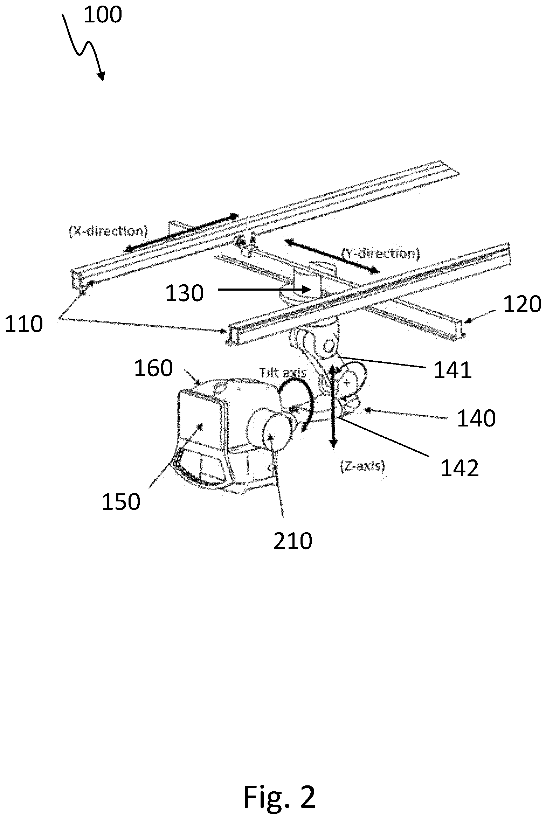

Fig. 2 shows a schematic setup of a ceiling-suspended holding system 100 for an X-ray tube head assembly 210 according to an embodiment of the invention. The holding system 100 comprises a stationary rail system 110 that is mounted to the ceiling of, for example, an X-ray examination room, and movable rail system 120 that is attached to the stationary rail system 100 such that it moves along the stationary rail system 110 along the horizontal x-direction. A carriage 130 is mounted to the movable rail system 120 such that it moves along the movable rail system 120 along the horizontal y-direction. A manipulator 140 like a robot arm is attached to the carriage 130 and comprising at least a first elongate member 141 and a second elongate member 142, which are serially interconnected by a first hinged joint 143. The first elongate member 141 is mounted with its upper end to the carriage 130, and the second elongate member 142 is mounted with its lower end to an X-ray tube head assembly 210. Advantageously, the first elongate member 141 is mounted via a second hinged joint 144 and a first rotational joint 146 to the carriage 130, while the second elongate member 142 is mounted via a third hinged joint 145 and a second rotational joint 147 to the X-ray tube head assembly 210. Thus, the holding system 100 with its manipulator 140 allows movement of the X-ray tube head assembly 210 along six axes.

Thus, this holding system can be used with diagnostic X-ray devices provided with an X-ray source in the X-ray tube head connected to the ceiling support. The whole structure of system provides the required degrees of freedom to X-ray tube head so that it can be moved in the examination room for various clinical applications. The X-ray tube head can be connected to end of the robotic arm and act as an end effector. The X-ray tube head assembly can be proved with a user interface 150 and a force torque sensor 160, which is explained in detail below.

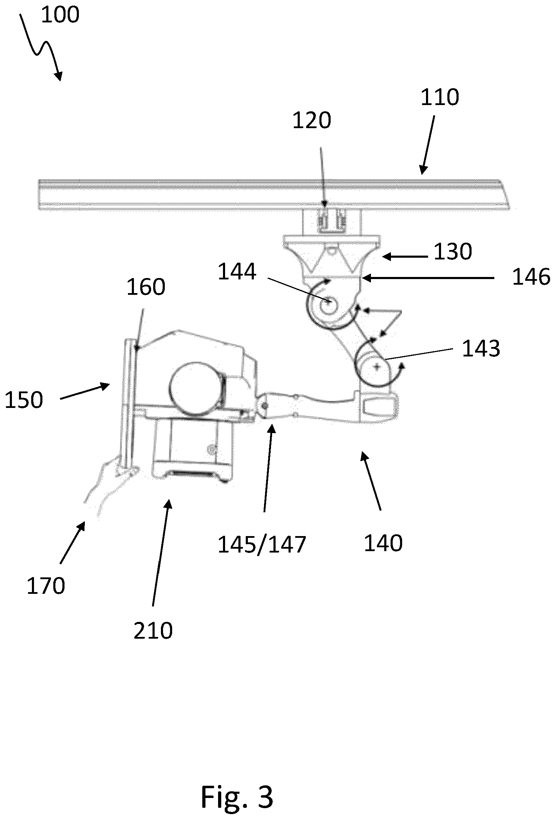

Fig. 3 shows a side view of a ceiling-suspended holding system 100 for an X-ray tube head assembly 210 according to an embodiment of the invention. The robotic arm of the manipulator can comprise several hardware elements like joints, sensors, motors, gearboxes, and drivers, each serving a specific function in enabling the arm movements along hinged or rotary movements for accurate positioning of the X-ray tube head for clinical examination. In particular, the manipulator comprises hinged joint 143, 144, 145, and rotary joints 146, 147.

For clinical examinations, there may be a user interface 150 at the front of X-ray tube head assembly 210 also known as control handle. Further, at each interface of relative motion in the robotic arm there may be electric motors with brakes and an optical encoder assembly. The user interface 150 may have switches which release a brake for motion in all, preferably five-axes. The robotic arm base may have roller bearings which allow the linear movement of suspended robotic arm on the transverse rail system. The whole transverse carriage in turn moves linearly on the longitudinal rail system via pairs of rollers.

Further, the robotic arm may have a mechanical emergency braking system for stopping the movements between the links in the case of a failure or incorrect control to avoid injury to patient or the user during the clinical workflows. Emergency braking can be triggered with overcurrent drawn by the motors due to increase in the resistance force during the collision.

If a user 170 applies a force to the X-ray head assembly 210, for example via a user interface 150, a force torque sensor 160 measures this force as positioning input of the user 170. Thus, a radiographer can interact with the holding system to reposition it. The force torque sensor can measure for example the force applied in six axes, for detecting a user force applied to the X-ray tube head assembly in different directions, and in turn can provide input how to reposition the holding system and the X-ray tube head assembly mounted thereto. A controller can receive this input and provide control signals to motors or actuators of the holding system for moving the joins of the manipulator and/or the rails systems.

Motor shafts in bearings with encoders can enable rotation around the specific axis. Similarly, hinged joints can be actuated between the interconnecting links or joints. Electric motors at each joint or interface can provide relative motion in at least five directions, and may also enable servo assistance corresponding to a user force applied to an interface like a control handle. The force torque sensor 160 between the user interface 150 and the X-ray tube head assembly 210 detects the user force in different directions and in turn give signal to motors of that axis movement.

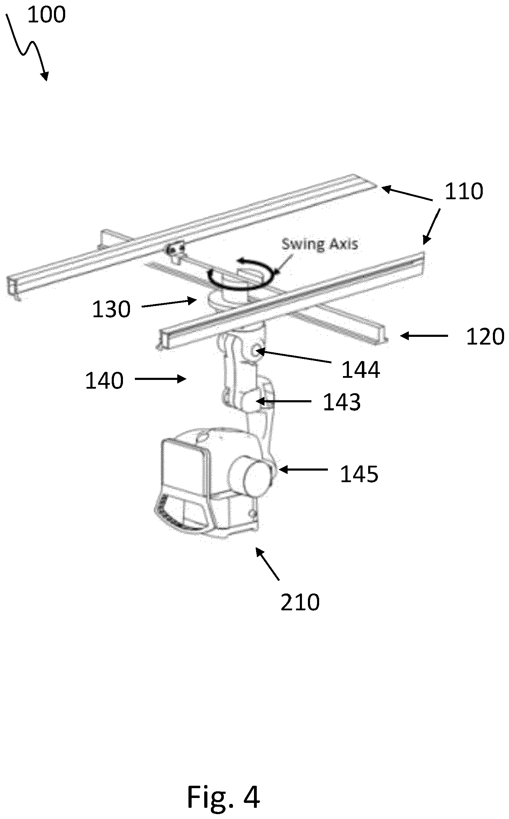

Fig. 4 shows a schematic setup of a ceiling-suspended holding system for an X-ray tube head assembly according to an embodiment of the invention. The first and second elongate members 141, 142 of the manipulator each comprise an unchangeable length, and in particular do not comprise any telescopic mechanism, which might change the length of any one of the serially interconnected members of the manipulator. Thus, the manipulator comprises as movable elements solely joints allowing a rotary movement around an axis. In particular, the manipulator comprises hinged joints and rotary joints, where the hinged joints connecting two members allow rotation about an axis essentially perpendicular to a connection direction of the two members, and where the rotary joints connecting two members allow rotation about an axis essentially parallel to a connection direction of the two members. Therefore, the holding system and/or the manipulator, with the exception of the rails systems, does not comprise any telescopic connectors, which allow a linear moment in any direction.

Movement of X-ray tube head assembly in the x- and y- direction may be initiated by disengaging the brakes between the rails and the carriage with the switch on the user interface. Now, the user can move the system in the desired direction. For motion in the swing and tilt axis, the brakes of rotary joints of the robotic arm may get disengaged with a switch on the user interface. Now, the user can rotate the X-ray tube head in the desired direction. Vertical motion of the X-ray tube head is possible due to the combined movement of hinged joint between serially interconnected supporting members of the robotic arm.

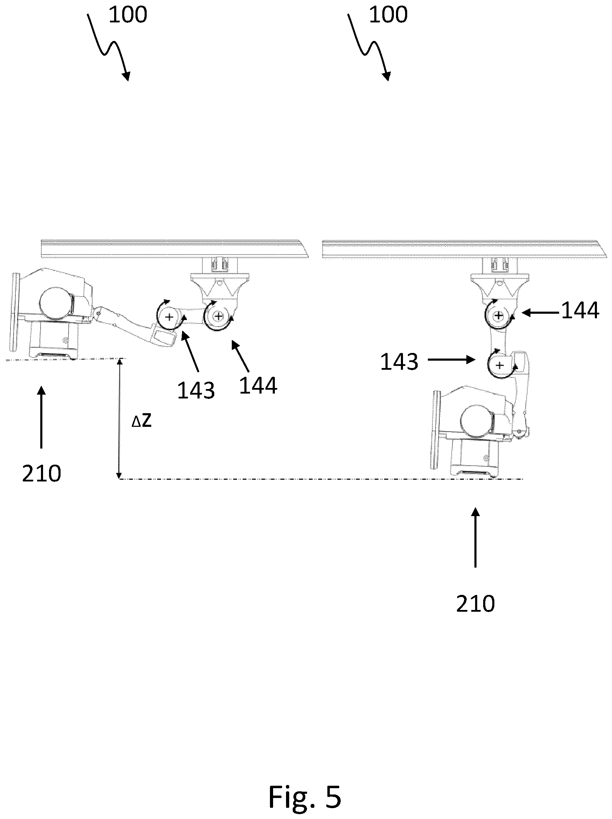

Fig. 5 shows two different configurations of a ceiling-suspended holding system 100 for an X-ray tube head assembly 210 according to an embodiment of the invention. By activation of a movement of the hinged joints 143 and 144 of the manipulator 140, a vertical movement of the X-ray tube head assembly 210 attached to the robotic arm can be effected, which results in a height difference Δz along the vertical z-axis.

The electrical cables, which are not shown, can be guided through chain/energy chain links which enable the horizontal movements of the carriage. These chains can be aligned in parallel with the fixed and moving ceiling rails. These chains for the vertical movement of X-ray tube head with heavy cables allow a comfortable handling of the holding system, which is otherwise not possible with traditional telescopic column systems. Multi-axis energy chains for the vertical movement provide unobstructed vertical movement. Complete replacement of conventionally used corrugated tubes is possible with a robotic arm for vertical movement. This advantageously gives an atheistically better look to the X-ray device and provides better experience to the user.

Therefore, an improved manipulator for the vertical movement of an X-ray tube is proposed. Currently, an energy chain for cable management is not used for vertical movement of an X-ray tube in commercially available products. The energy chain can advantageously be used in combination with a manipulator as proposed; thus, the present invention makes it possible to use the energy chain for cable management in the vertical direction, thereby avoiding the issue of the X-ray tube's hindrance during movement.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: ceiling-suspended holding system
- 110: stationary rails system
- 120: moving rail system
- 130: carriage
- 140: manipulator
- 141: first elongate member
- 142: second elongate member
- 143: first hinge
- 144: second hinge
- 145: third hinge
- 146: first rotary joint
- 147: second rotary joint
- 150: user interface
- 160: force torque sensor
- 170: user
- 210: X-ray tube head assembly

## Claims

1. A ceiling-suspended holding system (100) for an X-ray tube head assembly (210), the holding system (100) comprising:
a stationary rail system (110) configured to be mounted to a ceiling of an X-ray examination room;
a movable rail system (120) configured to be mounted to the stationary rail system (110), wherein the movable rail system (120) is configured to move along the stationary rail system (110) in a first direction;
a carriage (130) configured to be mounted to the movable rail system (120), wherein the carriage (130) is configured to move along the movable rail system (120) in a second direction; and
a manipulator (140) comprising at least a first elongate member (141) and a second elongate member (142),
wherein the first elongate member (141) is configured to be mounted with a first end of the first elongate member (141) to the carriage (130), and with a second end of the first elongate member (141) to a first end of the second elongate member (142), and wherein the second elongate member (142) is configured to be mounted with a second end of the second elongate member (142) to an X-ray tube head assembly (210); and
wherein the manipulator (140) comprises a first hinge (143) in-between the first elongate member (141) and the second elongate member (142).

2. The holding system (100) according to claim 1, wherein the holding system (100) comprises a second hinge (144) between the carriage (130) and the first elongate member (141) of the manipulator (140).

3. The holding system (100) according to any one of the preceding claims, wherein the holding system (100) comprises a first rotary joint (146) between the carriage (130) and the first elongate member (141) of the manipulator (140), the first rotary joint (146) configured for allowing a rotation of the manipulator (140) around a vertical axis.

4. The holding system (100) according to any one of the preceding claims, wherein the manipulator (140) comprises a second rotary joint (147) and/or a third hinge (145) between the second elongate member (142) of the manipulator (140) and the X-ray tube head assembly (210).

5. The holding system (100) according to any one of the preceding claims, wherein the first elongate member (141) comprises a first unchangeable length and the second elongate member (142) comprises a second unchangeable length.

6. The holding system (100) according to any one of the preceding claims, wherein the holding system (100) and/or the manipulator (140) does not comprise a telescopic mechanism.

7. The holding system (100) according to any one of the preceding claims, wherein the manipulator (140) comprises at least a third elongate member in-between the first and second elongate members.

8. The holding system (100) according to claim 7, wherein the third elongate member comprises a third unchangeable length.

9. The holding system (100) according to any one of the preceding claims, wherein the first direction is perpendicular to the second direction, and/or wherein the first direction and the second direction are parallel to the ceiling of the X-ray examination room.

10. The holding system (100) according to any one of the preceding claims, wherein the holding system further comprises an energy chain configured for guiding electrical cables from the carriage (130) along the manipulator (140) to the X-ray tube head assembly (210).

11. The holding system (100) according to any one of the preceding claims, wherein the stationary rail system (110) comprises a first stationary rail and a second stationary rail, wherein the first stationary rail is parallel to the second stationary rail.

12. The holding system (100) according to any one of the preceding claims, wherein the X-ray tube head assembly (210) is an X-ray source configured to be positioned movably in the X-ray examination room.

13. The holding system (100) according to any one of the preceding claims, wherein the holding system (100) comprises a user interface (150) and/or a force torque sensor (160) configured for receiving positioning input of a user (170), a controller, and at least one actuator configured for positioning the holding system (100) according to the positioning input of the user.

14. The holding system (100) according to any one of the preceding claims, further comprising a locking mechanism configured for locking the holding system (100) in a current position.

15. An X-ray device comprising
an X-ray tube head assembly (210),
an X-ray detector, and
a ceiling-suspended holding system (100) according to any one of claims 1 to 14.
